# EUROPÄISCHE PATENTSCHRIFT

(11) **EP 1 332 984 B1**
(45) Veröffentlichungstag und Bekanntmachung des Hinweises auf die Patenterteilung: **02.08.2006**
(21) Anmeldenummer: 03001140.7
(22) Anmeldetag: 21.01.2003
(51) Int. Cl.: B65D 83/06, A61M 15/00

(54) **Behälter zum Verpacken und Ausgeben von feinem Puder**
Container for the packaging and distribution of fine powders
Récipient pour le conditionnement et la distribution de poudres fines

(30) Priorität: 29.01.2002 DE 20201308 U
(43) Veröffentlichungstag der Anmeldung: 06.08.2003
(73) Patentinhaber: Filtertek B.V., County of Limerick (IE)
(72) Erfinder: Mijers, Jan Willem Marinus, 5913 TP Venlo (NL); Hogan, Brendan, Raheen, Co. Limerick (IE)
(74) Vertreter: Brose, D. Karl

(56) Entgegenhaltungen:
- DE-U- 20 201 308
- NL-A- 8 403 186
- US-A- 4 249 526

## Beschreibung

Die Erfindung betrifft einen Behälter zum Verpacken und Ausgeben von feinem Puder mit zwei Abteilen, welche durch ein Filtersieb voneinander getrennt sind, wobei das erste Abteil den Puder aufnimmt und die Ausgabe durch das Filtersieb hindurch über das zweite Abteil erfolgt, wobei ferner der Behälter aus einem oberen, das erste Abteil enthaltenden Gehäuseteil und einem, das zweite Abteil enthaltenden unteren Gehäuseteil besteht und die beiden aus Kunststoff bestehenden Gehäuseteile mit dem dazwischen angeordneten Filtersieb miteinander verbunden sind.

Derartige Behälter wurden für spezielle Zwecke entwickelt, in denen ein sehr feiner Puder in einen kleinen Behälter verpackt werden muss, und zwar in einer derartigen Weise, dass die Ausgabe des Puders durch den Boden des Behälters hindurch erfolgt, wobei der Boden ein sehr feines Filtersieb enthält. Hierbei wird Druckluft durch den aus Filtersieb bestehenden Boden des Behälters geblasen, so dass der feine Puder in den Auslass des gleichzeitig als Ausgabevorrichtung dienenden Behälters geblasen wird. Diese Art von Puderbehälter ist ein sehr wichtiger Teil der jeweils verwendeten Ausgabeeinrichtung.

Zum Herstellen derartiger Behälter ist es bekannt, diese mittels eines Einsatz-Spritzgiessverfahrens herzustellen. Dies führt jedoch bei den bekannten Behältern der oben definierten Art dazu, dass die Löcher in der Kante des Filtersiebes angrenzend an den Innendurchmesser des Behälters durch den Kunststoff beim Spritzgiessen verschlossen werden, welcher in diese Behälter eindringt. Beim Einsatzspritzgiessen wird dieses Problem noch dadurch verstärkt, dass die Kerne der Spritzgiessform auf das Filtersieb einen lokalen Druck ausüben, durch den an diesen Stellen der Fadendurchmesser des Siebs zusammenbricht und so zusätzliche Sieböffnungen schliesst. Dies ist jedoch für die meisten Anwendungsfälle derartiger Behälter unzulässig, da kleine Mengen des Puders während der Ausgabe am Rand zurückbleiben können. Insbesondere werden derartige Behälter zur Ausgabe von Medikamenten in Puderform verwendet, so dass hierbei die Ausgabe des puderförmigen Medikaments nicht vollständig ist, sondern ein Teil der in den überwiegenden Fällen ausgesprochen teuren Medikamente in dem Behälter verbleiben. Hierbei ist von besonderer Bedeutung, dass es bei derartigen Medikamenten oft auf eine sehr genaue Dosierung ankommt, welche nicht gewährleistet ist, wenn nicht genau festlegbare Mengen in dem Behälter zurückbleiben.

Der Erfindung liegt die Aufgabe zugrunde, einen Behälter der eingangs genannten Art zu schaffen, bei welchem trotz leichter Herstellbarkeit in verschiedenen Verfahrensvarianten dieses Problem vermieden wird.

Erfindungsgemäss wird bei einem Behälter der oben definierten Art diese Aufgabe im wesentlichen dadurch gelöst, dass die lichte Weite des das zweite Abteil enthaltenden unteren Gehäuseteils grösser ist als die lichte Weite des das erste Abteil enthaltenden oberen Gehäuseteils, derart, dass die Unterkante des oberen Gehäuseteils das Filtersieb an seinem Gesamtumfang überdeckt. Es ist offensichtlich, dass hierdurch eine Konstruktion geschaffen wird, bei welcher sichergestellt ist, dass an der Unterkante des ersten Abteils keine Löcher des Filtersiebs in irgendeiner Weise verschlossen sind, so dass eine vollständige Ausgabe des feinen Puders ohne Rückstände erfolgen kann.

Bei einer bevorzugten Ausführungsform sind die Gehäuseteile beide zylindrisch ausgebildet.

Im einzelnen kann die Erfindung dadurch weitergebildet werden, dass der obere Gehäuseteil an seiner Unterkante mit einem äusseren Flansch versehen ist.

Im einzelnen ist es hierbei von Vorteil, dass der untere Gehäuseteil an seiner Oberkante mit einem äusseren Flansch versehen ist, dessen Grösse der Grösse des am oberen Gehäuseteil vorgesehenen Flansches entspricht.

Bei einer besonders bevorzugten Ausführungsform nach der Erfindung ist am äusseren Flansch des oberen Gehäuseteils eine Ringschürze angeformt, welche in eine ringförmige Stufe im äusseren Flansch des unteren Gehäuseteils eingreift. Hierdurch wird die Herstellung wegen der selbstzentrierenden Bauweise der beiden Gehäuseteile stark erleichtert.

Besonders bevorzugt ist es ferner, dass das Filtersieb zunächst auf dem äusseren Flansch des unteren Gehäuseteils befestigt wird, wobei sich beispielsweise das Filtersieb einstückig mit dem unteren Gehäuseteil durch Einsatzspritzgiessen herstellen lässt.

Bevorzugt wird hierbei das Filtersieb innerhalb der ringförmigen Stufe angeordnet.

Gemäss einer besonders bevorzugten Herstellungsweise des erfindungsgemässen Behälters wird der obere Gehäuseteil getrennt von dem unteren Gehäuseteil durch Spritzgiessen hergestellt und der untere Gehäuseteil einschliesslich des Filtersiebs mit dem oberen Gehäuseteil durch Ultraschallschweissen an den Flanschen verbunden.

Durch die oben stehenden Massnahmen ist unabhängig von der Herstellungsweise sichergestellt, dass die Zone der Verbindung zwischen den beiden Gehäuseteilen gewährleistet, dass am Rand des ersten, den Puder enthaltenden Abteils keine Rückstände verbleiben können.

Im folgenden wird die Erfindung anhand einer in den Zeichnungen beispielhaft veranschaulichten Ausführungsform näher erläutert.

Es zeigt:
**FIGUR 1** eine schematische Schnittansicht eines derartigen Behälters, bei welchem der das erste von dem zweiten Abteil trennende Bereich nach dem Stand der Technik ausgebildet ist;
**FIGUR 2** eine Schnittansicht der beiden, den Behälter nach der Erfindung bildenden Gehäuseteile und
**FIGUR 3** eine Figur 1 entsprechende Schnittansicht eines Behälters nach der Erfindung.

In der folgenden Beschreibung wurden für gleiche bzw. gleichwirkende Teile jeweils gleiche Bezugszeichen verwendet.

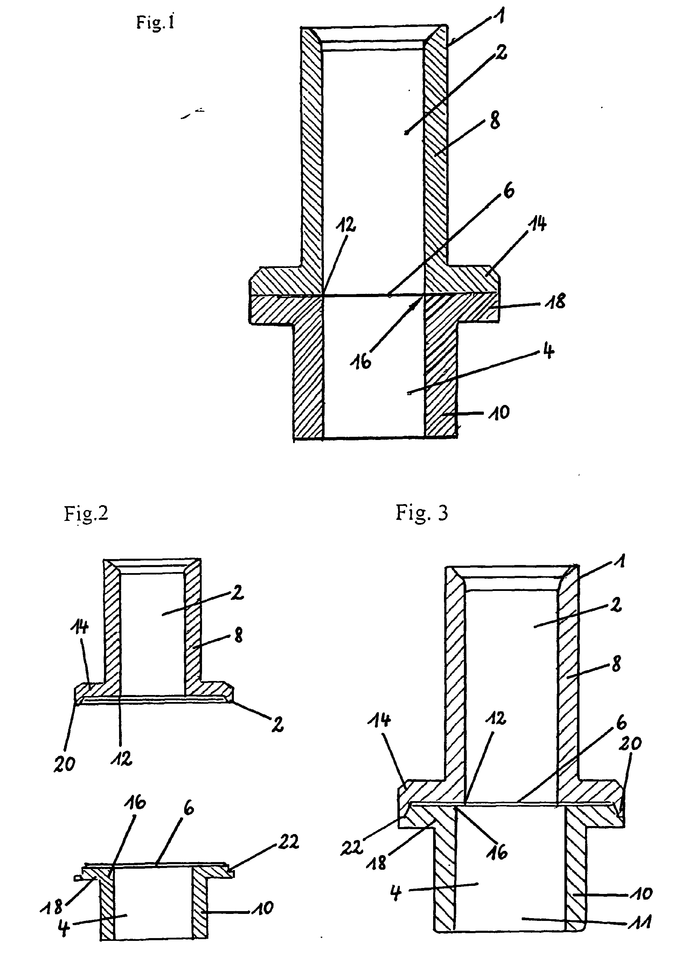

Die Schnittansicht gemäss Figur 1 zeigt einen Behälter 1, dessen Innenraum nach dem Stand der Technik ausgebildet ist.

Wie bereits erwähnt, wurden derartige Behälter für spezielle Zwecke entwickelt, bei denen ein sehr feiner Puder in den kleinen Behälter derart eingepackt ist, dass die Ausgabe des Puders durch den Boden des Behälters 1 hindurch erfolgt. Hierbei ist der Boden des Behälters 1 durch ein feines Filtersieb 6 gebildet, wobei Druckluft durch den aus dem Filtersieb 6 bestehenden Boden des Behälters hindurchgeblasen wird und der feine Puder in den Auslass 11 geblasen wird. Der Puderbehälter 1 stellt einen sehr wichtigen Teil der letztlich verwendeten Ausgabevorrichtung dar, wobei mehrere Verfahren zur Verfügung stehen, einen derartigen Behälter, dessen Innenraum gemäss Figur 1 ausgebildet ist, herzustellen. Üblicherweise werden derartige Behälter durch Einsatzspritzgiessverfahren hergestellt, was zwangsläufig zu dem Ergebnis führt, dass die Löcher im Randbereich des Filtersiebes 6 durch den Kunststoff verschlossen werden, in den das Filtersieb 6 eingegossen wird. Hierdurch können jedoch geringfügige Mengen des Puders während der Ausgabe, beispielsweise eines Medikaments in Puderform, im Behälter 1 zurückbleiben, so dass von dem meist teuren Medikament ein Rest im Behälter 1 verbleibt.

Wie in Figur 1 gezeigt, besteht ein derartiger Behälter 1, dessen Innenraum nach dem Stand der Technik ausgebildet ist, aus zwei Abteilen 2 und 4, welche durch das Filtersieb 6 voneinander getrennt sind. Das erste Abteil 2 enthält den auszugebenden Puder, welcher durch das Filtersieb 6 hindurch über das zweite Abteil 4 ausgegeben wird.

Der Behälter 1 besteht üblicherweise aus einem oberen, das erste Abteil 2 enthaltenden Gehäuseteil 8 und einem unteren, das zweite Abteil 4 enthaltenden Gehäuseteil 10. Die beiden aus Kunststoff bestehenden Gehäuseteile 8 und 10 mit dem dazwischen angeordneten Filtersieb 6 sind miteinander fest verbunden.

Es wird nunmehr auf die in den Figuren 2 und 3 veranschaulichte Ausführungsform nach der Erfindung bezug genommen.

Wie gezeigt, ist erfindungsgemäss die lichte Weite des das zweite Abteil 4 enthaltenden unteren Gehäuseteils 10 grösser als die lichte Weite des das erste Abteil 2 enthaltenden oberen Gehäuseteils 8. Hierdurch überdeckt die Unterkante 12 des oberen Gehäuseteils 8 das Filtersieb 6 an seinem gesamten Umfang geringfügig.

Wie gezeigt, sind die Gehäuseteile 8 und 10 zylindrisch ausgebildet und der obere Gehäuseteil 8 weist an seiner Unterkante 12 einen äusseren Flansch 14 auf.

Der untere Gehäuseteil 10 weist an seiner Oberkante 16 ebenfalls einen äusseren Flansch 18 auf, dessen Grösse der Grösse des am oberen Gehäuseteil 8 vorgesehenen Flansches 14 entspricht.

Am äusseren Flansch 14 des oberen Gehäuseteils 8 ist eine Ringschürze 20 angeformt, welche in eine ringförmige Stufe 22 im äusseren Flansch 18 des unteren Gehäuseteils 10 eingreift.

Bei der Herstellung des erfindungsgemässen Behälters 1 wird beispielsweise durch Einsatzspritzgiessen das Filtersieb 6 zunächst an dem unteren Gehäuseteil 10 befestigt, wie dies in der unteren Hälfte von Figur 2 dargestellt ist. Hierbei ist das Filtersieb innerhalb der ringförmigen Stufe 22 angeordnet.

Der obere Gehäuseteil 8 wird getrennt von dem unteren Gehäuseteil 10 ebenfalls durch Spritzgiessen hergestellt, wie dies in der oberen Hälfte von Figur 2 dargestellt ist.

Anschliessend wird der das Filtersieb 6 enthaltende untere Gehäuseteil 10 mit dem oberen Gehäuseteil 8 durch Ultraschallschweissen an den Flanschen 14 und 18 verbunden.

Das Einsatzspritzgiessen des unteren Gehäuseteils 10 zusammen mit dem Filtersieb 6 ist ein vollständig automatisierbares Verfahren, wobei die kleinen kreisförmigen Stücke des Filtersiebs in der Spritzgiessform ausgestanzt werden und der untere Gehäuseteil dann gegen die ausgestanzten Filtersiebstücke spritzgegossen werden. Bei dem Einsatzspritzgiessen des unteren Gehäuseteils 10 mit dem Filtersieb 6 ist unvermeidlich, dass durch den Kunststoff des unteren Gehäuseteils 10 einige der Öffnungen in der Kante des Filtersiebs geschlossen werden.

Bei dem Behälter 1 nach der vorliegenden Erfindung stellt dies jedoch kein Problem dar, da der Innendurchmesser des unteren Gehäuseteils 10 grösser ist als der Innendurchmesser des oberen Gehäuseteils 8, so dass der Bereich, in welchem Öffnungen in dem Filtersieb 6 durch den Kunststoff verschlossen sein können, durch die Unterkante 12 des oberen Gehäuseteils 8 überdeckt werden, da der obere Gehäuseteil 8 auf dem Filtersieb 6 ringsum an dessen Rand aufliegt. Folglich wird bei der Ausgabe des Puders aus dem ersten Abteil 2 in das zweite Abteil 4 das erste Abteil 2 vollständig entleert und es bleiben keine Reste in dem Behälter zurück.

Obwohl als bevorzugte Herstellungsweise das Einsatzspritzgiessen der aus Filtersieb bestehenden Scheiben in den unteren Gehäuseteil 10 beschrieben wurden, lässt sich der Behälter 1, dessen Innenraum nach der Erfindung ausgestaltet ist, selbstverständlich auch auf andere Art und Weise herstellen.

Beispielsweise kann man die beiden Gehäuseteile 8 und 10 getrennt voneinander im Spritzgiessen herstellen und die ausgestanzten Scheiben aus dem Filtersieb 6 durch Ultraschallschweissen zunächst an dem unteren Gehäuseteil 10 befestigen und danach die beiden Gehäuseteile 8 und 10 mit dem dazwischen angeordneten Filtersieb 6 ebenfalls durch Ultraschallschweissen miteinander verbinden.

Alternativ ist es auch möglich, die beiden Gehäuseteile 8 und 10 ebenfalls getrennt voneinander herzustellen, dann die Scheiben aus dem Filtersieb 6 dazwischen anzuordnen und alle drei Teile gleichzeitig durch Ultraschallschweissen miteinander zu verbinden.

Eine weitere Alternative besteht darin, die aus Filtersieb 6 bestehenden Scheiben durch Einsatzspritzgiessen mit einem Kunststoffring zu versehen, den Kunststoffring mit dem unteren Gehäuseteil 10 und dem oberen Gehäuseteil 8 durch Ultraschallschweissen zu verbinden. Auf jeden Fall ist durch den kleineren Durchmesser des das erste Abteil 2 enthaltenden oberen Gehäuseteils 8 immer gewährleistet, dass die Vorteile nach der Erfindung erzielt werden, indem bei der Ausgabe des Puders aus dem Behälter 1 keine Reste zurückbleiben.

### BEZUGSZEICHENLISTE

- 1 =: Behälter
- 2 =: erstes Abteil
- 4 =: zweites Abteil
- 6 =: Filtersieb
- 8 =: oberer Gehäuseteil
- 10 =: unterer Gehäuseteil
- 11 =: Auslass
- 12 =: Unterkante von 8
- 14 =: Flansch von 8
- 16 =: Oberkante von 10
- 18 =: Flansch von 10
- 20 =: Ringschürze
- 22 =: Stufe

## Patentansprüche

1. Behälter zum Verpacken und Ausgeben von feinem Puder mit zwei Abteilen, welche durch ein Filtersieb voneinander getrennt sind, wobei das erste Abteil den Puder aufnimmt und die Ausgabe durch das Filtersieb hindurch über das zweite Abteil erfolgt, wobei ferner der Behälter aus einem oberen, das erste Abteil enthaltenden Gehäuseteil und einem, das zweite Abteil enthaltenden unteren Gehäuseteil besteht und die beiden aus Kunststoff bestehenden Gehäuseteile mit dem dazwischen angeordneten Filtersieb miteinander verbunden sind, **dadurch gekennzeichnet, dass** die lichte Weite des das zweite Abteil (4) enthaltenden unteren Gehäuseteils (10) grösser ist als die lichte Weite des das erste Abteil (2) enthaltenden oberen Gehäuseteils (8) derart, dass die Unterkante (12) des oberen Gehäuseteils (8) das Filtersieb (6) an seinem Gesamtumfang überdeckt.

2. Behälter nach Anspruch 1, **dadurch gekennzeichnet, dass** die Gehäuseteile (8, 10) zylindrisch ausgebildet sind.

3. Behälter nach Anspruch 1 oder 2, **dadurch gekennzeichnet, dass** der obere Gehäuseteil (8) an seiner Unterkante (12) mit einem äusseren Flansch (14) versehen ist.

4. Behälter nach Anspruch 3, **dadurch gekennzeichnet, dass** der untere Gehäuseteil (10) an seiner Oberkante (16) mit einem äusseren Flansch (18) versehen ist, dessen Grösse der Grösse des am oberen Gehäuseteil (8) vorgesehenen Flansches (14) entspricht.

5. Behälter nach Anspruch 4, **dadurch gekennzeichnet, dass** an dem äusseren Flansch (14) des oberen Gehäuseteils (8) eine Ringschürze (20) angeformt ist, welche in eine ringförmige Stufe (22) im äusseren Flansch (18) des unteren Gehäuseteils (10) eingreift.

6. Behälter nach Anspruch 4 oder 5, **dadurch gekennzeichnet, dass** das Filtersieb (6) zunächst auf dem Flansch (18) des unteren Gehäuseteils (10) befestigt ist.

7. Behälter nach Anspruch 6, **dadurch gekennzeichnet, dass** das Filtersieb (8) einstückig mit dem unteren Gehäuseteil (10) durch Einsatzspritzgiessen hergestellt ist.

8. Behälter nach einem der Ansprüche 5 bis 7, **dadurch gekennzeichnet, dass** das Filtersieb (6) innerhalb der ringförmigen Stufe (22) angeordnet ist.

9. Behälter nach Anspruch 8, **dadurch gekennzeichnet, dass** der obere Gehäuseteil (8) getrennt von dem unteren Gehäuseteil (10) durch Spritzgiessen hergestellt ist, und dass der untere Gehäuseteil (10) einschliesslich des Filtersiebs (6) mit dem oberen Gehäuseteil (8) durch Ultraschallschweissen an den Flanschen (14, 18) verbunden ist.

## Claims

1. Container for packaging and dispensing of a fine powder having two compartments, which are separated by a filter mesh, wherein the first compartment is receiving the powder and the dispensing is done through the filter mesh via the second compartment, wherein further the container is consisting of an upper housing member containing the first compartment and a lower housing member containing the second compartment with the two housing members consisting of plastics are fixed to another with the filter mesh positioned there between, **characterised in that** the inside diameter of the lower housing member (10) containing the second compartment (4) is larger than the inside diameter of the upper housing member (8) containing the first compartment (2) such that the bottom edge (12) of the upper housing member (8) is covering the entire circumference of the filter mesh (6).

2. Container according to claim 1, **characterised in that** the housing members (8, 10) are cylindrical.

3. Container according to claim 1 or 2, **characterised in that** the upper housing member (8) is provided with an exterior flange (14) at the bottom edge (12).

4. Container according to claim 3, **characterised in that** the lower housing member (10) is provided with an exterior flange (18) at the upper edge (16), the size of which is corresponding to the size of the flange (14) provided at the upper housing member (8).

5. Container according to claim 4, **characterised in that** on the exterior flange (14) of the upper housing member (8) there is provided an annular skirt (20), which is engaging an annular step (22) in the exterior flange (18) of the lower housing member (10).

6. Container according to claim 4 or 5, **characterised in that** the filter mesh (6) firstly is fixed to the flange (18) of the lower housing member (10).

7. Container according to claim 6, **characterised in that** the filter mesh (8) is unitarily produced with the lower housing member (10) by insert-moulding.

8. Container according to any of the claims 5 to 7, **characterised in that** the filter mesh (6) is positioned within the annular step (22).

9. Container according to claim 8, **characterised in that** the upper housing member (8) is produced separately from the lower housing member (10) by injection moulding and **in that** the lower housing member (10) including the filter mesh (6) is connected with the upper housing member (8) at the flanges (14, 18) by ultrasonic welding.

## Revendications

1. Récipient pour le conditionnement et la distribution de poudres fines, comportant deux compartiments qui sont séparés l'un de l'autre par un tamis filtrant, le premier compartiment recevant la poudre et la distribution s'effectuant à travers le tamis filtrant par l'intermédiaire du deuxième compartiment, le récipient étant formé en outre par une partie de boîtier supérieure contenant le premier compartiment et par une partie de boîtier inférieure contenant le deuxième compartiment et les deux parties de boîtier, réalisées en matière plastique, sont assemblées l'une à l'autre avec le tamis filtrant agencé entre elles, **caractérisé en ce que** la largeur intérieure de la partie de boîtier inférieure (10), contenant le deuxième compartiment (4), est supérieure à la largeur intérieure de la partie de boîtier supérieure (8) contenant le premier compartiment (2), de telle sorte que le bord inférieur (12) de la partie de boîtier supérieure (8) couvre le tamis filtrant (6) sur tout son pourtour.

2. Récipient selon la revendication 1, **caractérisé en ce que** les parties de boîtier (8, 10) sont cylindriques.

3. Récipient selon la revendication 1 ou 2, **caractérisé en ce que** la partie de boîtier supérieure (8) est munie d'une bride extérieure (14) sur son bord inférieur (12).

4. Récipient selon la revendication 3, **caractérisé en ce que** la partie de boîtier inférieure (10) est munie, sur son bord supérieur (16), d'une bride extérieure (18), dont la dimension correspond à la dimension de la bride (14) prévue sur la partie de boîtier supérieure (8).

5. Récipient selon la revendication 4, **caractérisé en ce que** sur la bride extérieure (14) de la partie de boîtier supérieure (8) est formée une jupe annulaire (20) qui s'engage dans un décrochement (22) annulaire dans la bride extérieure (18) de la partie de boîtier inférieure (10).

6. Récipient selon la revendication 4 ou 5, **caractérisé en ce que** le tamis filtrant (6) est fixé d'abord sur la bride (18) de la partie de boîtier inférieure (10).

7. Récipient selon la revendication 6, **caractérisé en ce que** le tamis filtrant (6) est réalisé d'un seul tenant avec la partie de boîtier inférieure (10) par un procédé de moulage par injection.

8. Récipient selon l'une quelconque des revendications 5 à 7, **caractérisé en ce que** le tamis filtrant (6) est agencé à l'intérieur du décrochement (22) annulaire.

9. Récipient selon la revendication 8, **caractérisé en ce que** la partie de boîtier supérieure (8) est réalisée séparément de la partie de boîtier inférieure (10) par un procédé de moulage par injection, et **en ce que** la partie de boîtier inférieure (10), y compris le tamis filtrant (6), est assemblée à la partie de boîtier supérieure (8) par soudage aux ultrasons au niveau des brides (14, 18).
